# EUROPÄISCHE PATENTSCHRIFT

(11) **EP 1 503 670 B1**
(45) Veröffentlichungstag und Bekanntmachung des Hinweises auf die Patenterteilung: **30.04.2008**
(21) Anmeldenummer: 03752702.5
(22) Anmeldetag: 16.05.2003
(51) Int. Cl.: A61B 5/11, A61B 5/0476, G01S 13/58

(54) **VERFAHREN UND VORRICHTUNG ZUM AUTOMATISCHEN ERFASSEN MOTORISCHER UNRUHE EINER VERSUCHSPERSON**
METHOD AND DEVICE FOR THE AUTOMATIC DETECTION OF MOTORY DISTURBANCES IN A TEST PERSON
PROCEDE ET DISPOSITIF DE DETECTION AUTOMATIQUE DE L'AGITATION MOTRICE D'UN SUJET TESTE

(30) Priorität: 16.05.2002 DE 10221839
(43) Veröffentlichungstag der Anmeldung: 09.02.2005
(73) Patentinhaber: Universitätsklinikum Charité Medizinische Fakultät der Humboldt-Universität zu Berlin, 10117 Berlin (DE)
(72) Erfinder: HUSS, Michael, 14532 Kleinmachnow (DE); JENETZKY, Ekkehart, 12163 Berlin (DE)
(74) Vertreter: Bittner, Thomas L.
(86) Internationale Anmeldenummer: PCT/DE2003/001588
(87) Internationale Veröffentlichungsnummer: WO 2003/096902

(56) Entgegenhaltungen:
- EP-A- 0 064 788
- EP-A- 0 608 948
- US-A- 4 112 926
- BUTTE N ET AL.: "Stimulant medications decrease energy expenditure and physical activity in children with attention-deficit/hyperactivity disorder" THE JOURNAL OF PEDIATRICS, Bd. 135, Nr. 2 pt 1, - August 1999 (1999-08) Seiten 203-207, XP008021582
- SNITKER S ET AL.: "Spontaneous physical activity in a respiratory chamber is correlated to habitual physical activity" INTERNATIONAL JOURNAL OF OBESITY, Bd. 25, Nr. 10, - Oktober 2001 (2001-10) Seiten 1481-1486, XP002253395
- KROPVELD D ET AL: "DOPPLER RADAR DEVICE AS A USEFUL TOOL TO QUANTIFY THE LIVELINESS OFTHE EXPERIMENTAL ANIMAL" MEDICAL AND BIOLOGICAL ENGINEERING AND COMPUTING, PETER PEREGRINUS LTD. STEVENAGE, GB, Bd. 31, Nr. 4, 1. Juli 1993 (1993-07-01), Seiten 340-342, XP000399316 ISSN: 0140-0118

## Beschreibung

Die Erfindung betrifft ein Verfahren und eine Vorrichtung zum automatischen Erfassen motorischer Unruhe einer Versuchsperson.

Eine der häufigsten kinderpsychatrischen Störungen ist die sogenannte ADHD (ADHD-"Attention Defecit Hyperactive Disorder"), welche Störungen der Aufmerksamkeit, der Impulskontrolle sowie motorische Unruhe der Kinder umfaßt. Im Rahmen der Diagnostik der ADHD werden gegenwärtig überwiegend subjektive Fragebogenverfahren eingesetzt, bei denen die von der ADHD betroffenen Personen und/oder angehörige befragt werden.

Zur Objektivierung der Diagnoseverfahren wurde die Nutzung von beschleunigungsempfindlichen Bewegungsmessern (Akzellerometer) genutzt. Hierbei werden am Körper der Versuchsperson mehrere Meßelemente angebracht, mit denen Bewegungen von Körperteilen erfaßt werden können. Das Anbringen der Meßelemente erfordert einen direkten Körperkontakt bei der Versuchsperson, was bei der Versuchsperson zu Reaktionen führen kann, die ihr übliches Bewegungsverhalten verändern. Hierdurch wird die Auswertbarkeit der Versuchsergebnisse wesentlich eingeschränkt. Verfahren auf der Basis der Akzellerometer haben sich in der Routinediagnostik deshalb nicht durchsetzen können.

Verfahren auf der Basis von Elektromyographie (EMG) erfassen nur elektrische Muskelaktivität, die nicht zwingend mit tatsächlicher Bewegung gleichzusetzen ist. Da nicht jedes Zukken einer Muskelgruppe auch eine Bewegung eines Körperteils zur Folge hat, werden die Meßergebnisse hierdurch verfälscht. Daher eigenen sich auch diese Verfahren nicht zur Diagnostik des ADHD.

Bei einem weiteren bekannten Verfahren zum Erfassen motorischer Unruheereignisse bei einer Versuchsperson werden am Körper der Versuchsperson optische Reflektoren angebracht. Mit Hilfe einer Sendeeinrichtung werden dann Lichtsignale in Richtung der Versuchsperson ausgesendet. Es wird versucht, daß von den Reflektoren am Körper der Versuchsperson reflektierte Licht auszuwerten, um Informationen über motorische Unruhe der Versuchsperson zu gewinnen. Hierbei besteht ebenfalls der bereits beschriebene Nachteil, daß die am Körper der Versuchsperson angebrachten Reflektoren die übliche Bewegung der Versuchsperson beeinflussen. Darüber hinaus ist das Erfassen des reflektierten Lichts von den verschiedenen Reflektoren am Körper der Versuchsperson nur mit einem hohen gerätetechnischen Aufwand möglich.

Butte et al.: "Stimulant medications decrease energy expenditure and physical activity in children with attention-deficit/hyperactivity disorder", The Journal of Pediatrics, Bd. 135, Nr. 2 pt 1, August 1999, Seiten 203 - 207, beschreiben ein Verfahren und ein Gerät zum Erfassen motorischer Unruhe einer Versuchsperson, wobei in Einminuten-Intervallen die Aktivität der Versuchsperson mittels eines Doppler-Mikrowellen-Detektors gemessen wird.

Snitker et al.: "Spontaneous physical activity in a respiratory chamber is correlated to habitual physical activity", International Journal of Obesity, Bd. 25, Nr. 10, Oktober 2001, Seiten 1481 - 1486, offenbaren ein Verfahren zum Messen der Aktivität einer Versuchsperson mittels Mikrowellen-Bewegungssensoren. Es wird die Häufigkeit in einem vorbestimmten Zeitraum ermittelt.

Kropveld et al.: "Doppler radar device as a useful tool to quantify the liveliness of the experimental animal", Medical and Biological Engineering and Computing, Peter Peregrinus Ltd. Stevenage, GB, Bd. 31, Nr. 4, 1. Juli 1993, Seiten 340 - 342, offenbaren ein Doppler-Radar-Modul zum Messen einer Körperunruhe eines Tieres, wobei die Häufigkeit von Unruheereignissen ermittelt wird.

Aufgabe der Erfindung ist es, ein verbessertes Verfahren und eine verbesserte Vorrichtung der eingangs genannten Art anzugeben, bei dem (der) auf einfache Weise eine zuverlässige automatische Erfassung motorischer Unruheereignisse einer Versuchsperson so ermöglicht ist, daß beim Ausführen des Verfahrens und dem Anwenden der Vorrichtung eine Beeinflussung der üblichen Bewegungen in der Versuchsperson vermieden ist.

Diese Aufgabe wird erfindungsgemäß durch ein Verfahren nach dem unabhängigen Anspruch 1 und eine Vorrichtung nach dem unabhängigen Anspruch 8 gelöst.

Die Erfindung umfaßt insbesondere den Gedanken, Mikrowellensignale zum automatischen Erfassen motorischer Unruheereignisse einer Versuchsperson zu verwenden. Hierbei wird die Frequenz-Dopplerverschiebung, die zwischen ausgesendeten Mikrowellensignalen und empfangenen Mikrowellensignalen, welche an der Versuchsperson reflektiert wurden, auftreten, als Indikator für das Vorhandensein eines motorischen Unruheereignisses genutzt. Die Verwendung des Doppler-Effekts in Verbindung mit Mikrowellen hat gegenüber dem Stand der Technik den wesentlichen Vorteil, daß die motorischen Unruheereignisse unabhängig vom Anbringen spezieller Meßeinrichtungen oder Reflektoren am Körper der Versuchsperson erfaßt werden können. Auf diese Weise wird eine weitgehende Objektivierung der Erfassung der motorischen Unruheereignisse erreicht. Die Objektivierung der Messung verbessert die Diagnosemöglichkeiten sowohl beim Feststellen von psychiatrischen Störungen der Versuchsperson, die mit motorischer Unruhe verbunden ist, als auch beim Überprüfen der Wirkung einer Medikamenteneinnahme nach dem Diagnostizieren einer solchen Störung.

Darüber hinaus ermöglicht das Erfassen jeglicher motorischer Unruheereignisse der Versuchsperson, die sich im Sendekegel der ausgesendeten Mikrowellensignale befindet, im Vergleich zu den bekannten Versuchsmethoden mit Hilfe der Akzellerometer eine höhere Genauigkeit der Meßerfassung, da das "Zucken" einzelner Muskelgruppen nicht fälschlicher Weise als ein motorisches Unruheereignis registriert wird.

Die Erfindung hat weiterhin den Vorteil, daß mit Hilfe technischer Mittel erstmalig Unruheereignisse erfaßt werden, die von einem beliebigen Körperteil der Versuchsperson stammen können. Auf diese Weise ist die berühungslose Messung objektiv global quantifizierbar. Für Routineverfahren ist so eine Möglichkeit geschaffen, subjektive Einflüsse zu vermeiden, die bei den heute üblicherweise verwendeten Fragebögen eine wesentliche Rolle spielen.

Zumindest während eines Teils des vorbestimmten Zeitraums wird mit Hilfe einer Hirnstrom-Meßvorrichtung eine Hirnstrommessung bei der Versuchsperson ausgeführt, wobei von der Hirnstrom-Meßvorrichtung erfaßte Hirnstromsignale automatisch mit den empfangenen reflektierten Mikrowellensignalen sowie den ermittelten Frequenz-Dopplerverschiebungen elektronisch zeitlich korreliert werden. Die definierten Meßbedingungen für eine Himstrom-Messung schaffen auch für die Erfassung der Unruheereignisse Meßparameter, welche die Vergleichbarkeit der Meßergebnisse für unterschiedliche Messungen gewährleisten.

Eine zweckmäßige Weiterbildung des neuen Verfahrens sieht vor, daß automatisch zeitlich korreliert zu dem Aussenden der Mikrowellensignale mit Hilfe einer Entfernungsmeßeinrichtung wiederholt eine Messung der Entfernung zwischen der Versuchsperson und der Entfernungsmeßeinrichtung und/oder Objekten im Bereich des Sendekegels und der Entfernungsmeßeinrichtung ausgeführt wird. Auf diese Weise können beim Erfassen der motorischen Unruhe der Versuchsperson fehlerhafte Meßsignale infolge anderer bewegter Objekte, beispielsweise einer weiteren Person, im Bereich des Sendekegels eliminiert werden, wenn beim Erfassen einer Frequenz-Dopplerverschiebung infolge einer Bewegung der Versuchsperson zeitlich korreliert eine Entfernung festgestellt wird, die dem üblichen Abstand zwischen der Versuchsperson und der Entfernungsmeßeinrichtung nicht entspricht.

Eine mit Hilfe einfacher Mittel ausführbare und eine Schädigung der Versuchsperson ausschließende Möglichkeit zur Entfernungsmessung ist bei einer zweckmäßigen Ausgestaltung der Erfindung dadurch erreicht, daß zum Messen der Entfernung mit Hilfe der Entfemungsmeßeinrichtung weitere Mikrowellensignale oder optische Signale ausgesendet und reflektierte Teile der ausgesendeten weiteren Mikrowellensignale/optische Signale empfangen und ausgewertet werden.

Zur weiteren Objektivierung und der diskriminierenden Wirkung der Meßergebnisse kann bei einer vorteilhaften Ausführungsform der Erfindung vorgesehen sein, daß die reflektierten Mikrowellensignale während des vorbestimmten Zeitraums im Verlauf von Ruhephasen und von stimulierten Aktivitätsphasen der Versuchsperson erfaßt werden, wobei elektronische Informationen über einen Beginn und eine Ende der Ruhephasen und der stimulierten Aktivitätsphasen von der Auswertevorrichtung automatisch erfaßt werden.

Eine mit einfachen Mitteln ausführbare Möglichkeit zum Stimulieren der Versuchsperson ist bei einer bevorzugten Fortbildung der Erfindung dadurch erreicht, daß zum Stimulieren der Aktivitätsphasen Lichtsignale mit Hilfe einer Fotostimulationslampe ausgesendet werden.

Eine zweckmäßige Ausgestaltung der Erfindung kann vorsehen, daß mit Hilfe der Auswertevorrichtung unter Berücksichtigung der zeitlichen Korrelation mittels der erfaßten Hirnstromsignale eine Fehlerkorrektur der ermittelten Häufigkeit des Auftretens der motorischen Unruheereignisse in dem vorbestimmten Zeitraum automatisch ausgeführt wird. Beim Auftreten bestimmter Hirnstromsignale, die Reaktionen der Versuchsperson zugeordnet werden können, welche nicht mit einer motorischen Unruhebewegung der Versuchsperson verbunden sind, können die zeitgleich zu diesen Hirnstromsignalen erfaßten Frequenz-Dopplerverschiebungen als Indikatoren für motorische Unruheereignisse automatisch ausgeschlossen werden.

Bei einer bevorzugten Ausgestaltung der Erfindung ist vorgesehen, daß mit Hilfe der Auswertevorrichtung unter Berücksichtigung der zeitlichen Korrelation mit Hilfe der ermittelten Frequenz-Dopplerverschiebungen eine automatische Fehlerkorrektur der erfaßten Himstromsignale ausgeführt wird.

Die abhängigen Vorrichtungsansprüche weisen die in Verbindung mit dem jeweils zugehörigen abhängigen Verfahrensanspruch genannten Vorteile entsprechend auf.

Die Erfindung wird im folgenden anhand eines Ausführungsbeispiels unter Bezugnahme auf eine Zeichnung näher erläutert. Hierbei zeigen:
- Figur 1: eine schematische Darstellung einer Anordnung zum Erfassen motorischer Unruheereignisse einer Versuchsperson;
- Figur 2: eine grafische Darstellung einer beispielhaften Signalkurve; und
- Figur 3: eine grafische Darstellung der gemittelten Häufigkeit motorischer Unruheereignis-Signale bei Versuchspersonen verschiedenen Alters.

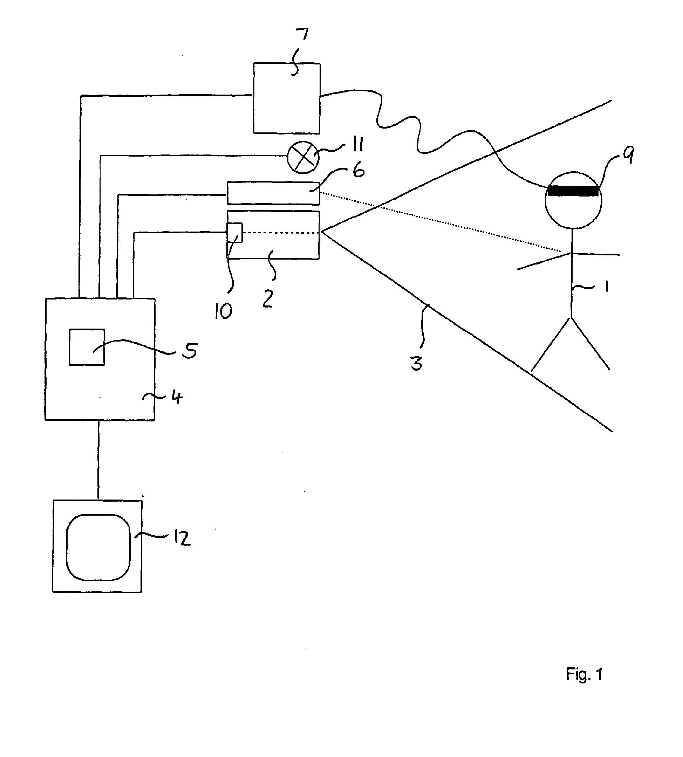

Figur 1 zeigt eine schematische Darstellung einer Anordnung zum automatischen Erfassen motorischer Unruheereignisse für eine Versuchsperson 1. Mit Hilfe einer Sende-/Empfangseinrichtung 2 werden Mikrowellensignale in einem Sendekegel 3, in dem sich die Versuchsperson 1 befindet, ausgesendet. Ausgesendete Mikrowellen, die an der Versuchsperson 1 reflektiert werden, können mit Hilfe der Sende-/Empfangseinrichtung 2 als reflektierte Mikrowellen empfangen werden. Die Sende-/Empfangseinrichtung 2 ist vorzugsweise als ein kombiniertes Sende-/Empfangsgerät (Transceiver) ausgebildet, es können jedoch auch getrennte Vorrichtungen zum Senden und Empfangen von Mikrowellen vorgesehen sein.

Wenn ein reflektiertes Mikrowellensignal an einem Körperteil der Versuchsperson reflektiert wurde, welches eine Bewegung ausführt, so weist das reflektierte Mikrowellensignal im Vergleich zu den ausgesendeten Mikrowellensignalen eine Doppler-Frequenzverschiebung auf, die eine Folge der Relativbewegung zwischen dem Körperteil und der Sende-/Empfangseinrichtung 2 ist. Für alle empfangenen, reflektierten Mikrowellensignale wird mit Hilfe einer Doppler-Auswerteeinrichtung 10 die Doppler-Frequenzverschiebung gegenüber den ausgesendeten Mikrowellensignalen erfaßt. Bei der Ausführungsform nach Figur 1 ist Doppler-Auswerteeinrichtung 10 in der Sende-/Empfangseinrichtung 2 angeordnet. Das dem beschriebenen Verfahren zugrunde liegende Meßprinzip des Doppler-Radars ist als solches wohlbekannt und wird in verschiedenen Doppler-Radargeräten genutzt, weshalb das Meßprinzip hier nicht weitergehend erläutert wird.

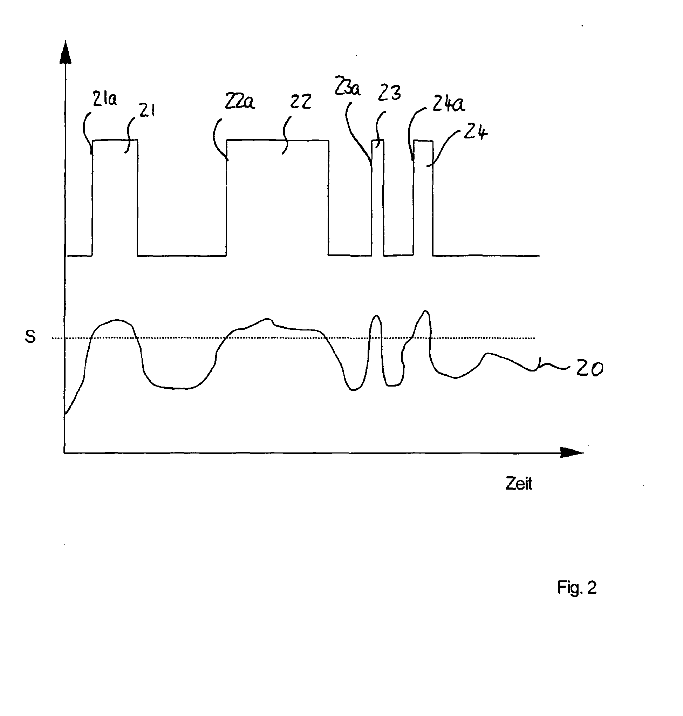

Figur 2 zeigt eine grafische Darstellung einer beispielhaften Meßkurve 20 für den Verlauf der in einem vorgegebenen Meßzeitraum kontinuierlich gemessenen Doppler-Frequenzverschiebung der empfangenen Mikrowellensignale. Die Information über die Doppler-Frequenzverschiebung der empfangenen Mikrowellensignale wird von der Sende-/Empfangseinrichtung 2 an eine Auswerteeinrichtung 4 (vgl. Figur 1) übertragen. Mit Hilfe der Auswerteeinrichtung 4, die eine Mikroprozessoreinrichtung 5 zum Verarbeiten der übermittelten elektronischen Informationen zur Doppler-Frequenzverschiebung aufweist, wird die gemessene Kurve 20 zum Erzeugen digitalisierter Signale nach dem Prinzip einer Analog-Digital-Wandlung verarbeitet. Gemäß Figur 2 werden Rechteckimpulse 21, 22, 23 und 24 stets dann erzeugt, wenn die Kurve 20 einen Schwellwert S überschreitet, wobei der Schwellwert S einer vorgegebenen Doppler-Frequenzverschiebung entspricht, bei der ein tatsächliches motorisches Unruheereignis angenommen wird.

Bei einer anschließenden Ermittlung der Häufigkeit des Auftretens motorischer Unruheereignisse wird mit Hilfe der Steuer- und Auswertevorrichtung 4 die Anzahl ansteigender Flanken 21a, 22a, 23a und 24a der Rechteckimpulse 21, 22, 23 und 24 automatisch ermittelt. Zu diesem Zweck werden die elektronischen Ergebnisse der Analog-Digital-Wandlung analysiert. Die Häufigkeit der so ermittelten motorischen Unruheereignisse innerhalb des vorgegebenen Meßzeitraums liefert Informationen für die Diagnostik der Versuchsperson, beispielsweise bezüglich des Vorliegens einer ADHD (ADHD-"Attention Deficit Hyperactive Disorder". Für Diagnoserückschlüsse kann der für eine Versuchsperson ermittelte Häufigkeitswert mit Kalibrierungskurven verglichen werden, die für eine große Anzahl von Versuchspersonen ermittelt wurden.). Mit Hilfe eine Vergleichs der Häufigkeitsmeßergebnisse für eine Versuchsperson zu verschiedenen Zeitpunkten kann bei der Versuchsperson auch die Wirkung einer Medikamenteneinnahme analysiert werden.

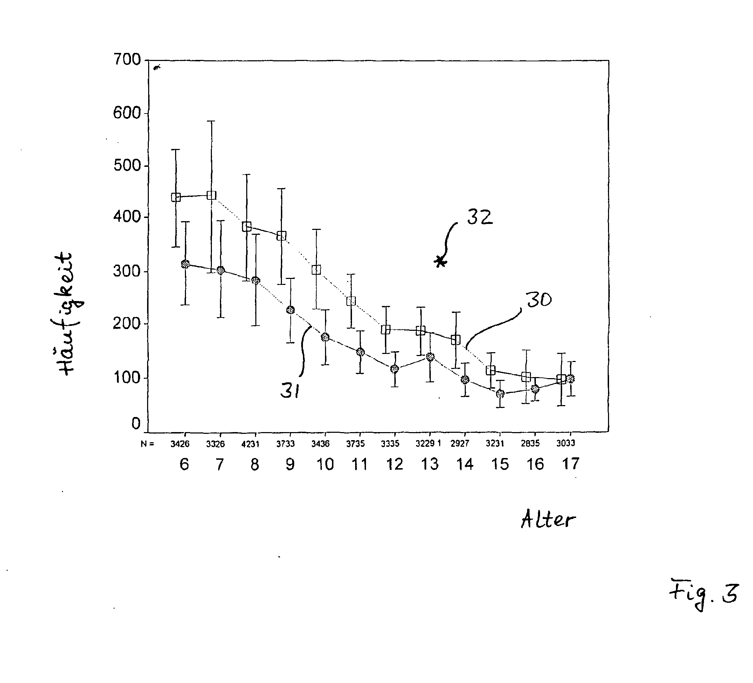

Figur 3 zeigt eine grafische Darstellung von Durchschnittswerten der ermittelten Häufigkeit motorischer Unruheereignisse für männliche (30) und weibliche (31) Versuchspersonen in Abhängigkeit vom Alter der Versuchsperson. Oberhalb des Werts für das Alter entlang der x-Achse ist die Anzahl N der Versuchspersonen angegeben, für die die Meßergebnisse zur Ermittlung der dargestellten Durchschnittswerte einbezogen wurden. Ein Meßpunkt 32 liegt weit oberhalb der Durchschnittswerte für eine Versuchsperson mit einem Alter von 13 Jahren, so daß in diesem Fall deutliche Anzeichen existieren, daß eine ADHD vorliegt.

Es werden im Folgenden weitere Details der Anordnung nach Figur 1 beschrieben. Ein wesentliches Element der Sende-/Empfangseinrichtung 2 ist ein als Sender genutzter Hochfrequenz-Osszillator, der im Mikrowellenbereich von 9.35 GHz arbeitet. Die abgestrahlte Gesamtleistung der Sendeeinrichtung beträgt etwa 1 mW, kann jedoch für den jeweiligen Anwendungsfall angepaßt werden, um einerseits eine ausreichendes Signal-Rausch-Verhältnis zu erreichen und andererseits eine Schädigung der Versuchsperson durch Mikrowellenbestrahlung mit Sicherheit auszuschließen. In Verbindung mit Mikrowellenherden werden 5 mW pro cm² als völlig unbedenklich für den Menschen eingestuft. Bei der verwendeten Sende-/Empfangseinrichtung 2 beträgt die Ansprechgrenze für Bewegungen der Versuchsperson 1 mindestens 6 cm/s bis maximal 80 km/h. Eine weitergehende Optimierung zum Erhöhen der Empfindlichkeit der Sende-/Empfangseinrichtung 2 kann vorgesehen sein, würde unter Umständen jedoch die Kosten zum Herstellen der Sende-/Empfangseinrichtung 2 erhöhen.

Der verwendete Hochfrequenzosszillator weist beispielsweise eine keulenförmige Abstrahlungscharakteristik von etwa +/-20 Grad auf. Die Empfangseinrichtung erfaßt jedoch einen Öffnungswinkel von etwa +/-60 Grad bis in eine Entfernung von etwa 5 bis 8 m. Üblicherweise befindet sich die Versuchsperson 1 in einer Entfernung von 1-2 m von der Sende-/Empfangseinrichtung 2. Diese Parameter können in Abhängigkeit vom Anwendungsfall an verschiedene Umgebungsbedingungen angepaßt werden. Hierzu stehen dem Fachmann die Erfahrungen im Bereich der als solchen bekannten Mikrowellensensorik zur Verfügung.

Gemäß Figur 1 ist weiterhin ein Entfernungsmesser 6 vorgesehen. Hierbei kann es sich einen beliebigen Entfernungsmesser handeln, der als solcher bekannt ist und für die Verwendung bei Versuchen mit Lebewesen, insbesondere wegen der verwendeten Strahlungsquelle, geeignet ist. Vorzugsweise wird ein Entfernungsmesser mit einer optischen Strahlungsquelle genutzt, die eine geringe Strahlungsintensität aufweist. Mit Hilfe des Entfernungsmessers 6 wird während des vorgegebenen Meßzeitraums der Abstand zwischen den Entfernungsmesser 6 und der Versuchsperson und/oder anderen Objekten im Bereich des Sendekegels 3 gemessen. Die Ergebnisse der Entfernungsmessung werden von dem Entfernungsmesser 6 an die Auswerteeinrichtung 4 übertragen, so daß in Verbindung mit Meßreihen, die mit Hilfe der Sende-/Empfangseinrichtung 2 aufgenommen werden, jeweils zugehörige Entfernungsmeßwerte als zusätzliche Meßinformation zur Verfügung stehen. Beim Vergleich von unterschiedlichen Meßreihen können die Entfernungsmeßwerte so als zusätzliches Auswerte- oder Vergleichskriterium herangezogen werden, beispielsweise um eine entfernungsabhängige Meßwerterfassung der Sende-/Empfangseinrichtung 2 zu berücksichtigen.

Mit Hilfe der Ergebnisse der Entfernungsmessung können beim Auswerten der digitalisierten Signale 21-24 (vgl. Figur 2) Signale als Fehler automatisch aussortiert werden, wenn zum Zeitpunkt des Signals mit Hilfe des Entfernungsmessers 6 ein vom üblichen Abstand zwischen dem Entfernungsmesser 6 und der Versuchsperson 1 abweichender Abstand gemessen wurde, was zu einer Stetigkeitsabweichung führt und auf ein anderes Objekt als die Versuchsperson 1 hindeutet. Hierbei kann es sich beispielsweise um eine die Messung kontrollierende Person handeln, die zwischen dem Entfernungsmesser 6 und der Versuchsperson 1 hindurchläuft. Um die gemessenen Entfernungssignale den digitalisierten Signalen 21-24 zuordnen zu können, müssen die Sende-/Empfangseinrichtung 2 und der Entfernungsmesser 6 auf einer gemeinsamen Zeitbasis arbeiten. Dieses kann beispielsweise mit Hilfe eines gemeinsamen Zeitgebers (nicht dargestellt) in der Auswerteeinrichtung 4 oder in einem der beiden Geräte, nämlich der Sende-/Empfangseinrichtung 2 oder dem Entfernungsmesser 6, sichergestellt werden. Bei dem Zeitgeber kann es sich beispielsweise um einen üblichen elektronischen Taktgeber handeln, wie er für die Erzeugung von Zeittakten bei gepulsten Messungen genutzt wird. Es ist darauf hinzuweisen, daß die Messung der motorischen Unruheereignisse an der Versuchsperson 1 mit Hilfe der Sende-/Empfangseinrichtung 2 grundsätzlich unabhängig von den Messungen mit Hilfe des Entfernungsmessers 6 ausgeführt werden kann. Die Nutzung des Entfernungsmessers 6 dient jedoch der Verminderung der Anzahl fehlerhafter Signale, die fälschlicherweise als motorische Unruheereignisse interpretiert werden.

Des weiteren ist gemäß Figur 1 eine Hirnstrom-Meßvorrichtung 7 an die Steuer- und Auswertevorrichtung 4 gekoppelt. Mit Hilfe der Hirnstrom-Meßeinrichtung 7 wird ein Standard-EEG (EEG - Elektroenzephalographie) aufgenommen. Dazu ist die Hirnstrom-Meßvorrichtung 7 über eine oder mehrere Leitungen 8 mit Elektroden 9 verbunden, die am Körper der Versuchsperson 1 angeordnet sind. Eine zeitliche Korrelierung der mit Hilfe Hirnstrom-Meßeinrichtung 7 gemessenen Hirnströme mit den empfangenen, reflektierten Mikrowellensignalen bzw. den hieraus ermittelten Doppler-Frequenzverschiebungen wird in der Auswerteeinrichtung 4 durchgeführt. Hierdurch ist es möglich, bei der Messung der Hirnströme mit Hilfe der Hirnstrom-Meßeinrichtung 7 und der Messung der motorischen Unruheereignisse mit Hilfe der Sende-/Empfangseinrichtung 2 gegenseitig fehlerhafte Signale auszuschließen. Dieses wird in der Auswerteeinrichtung 4 mit Hilfe einer automatischen Fehlerkorrektur durchgeführt. So können auf diese Weise beispielsweise einzelne Signale der digitalisierten Signale 21-24 als Indikatoren für ein motorisches Unruheereignis ausgeschlossen werden, wenn aus dem Verlauf der gemessenen Hirnströme festgestellt wird, daß eine Bewegung eines Körperteils der Versuchsperson 1 nicht stattgefunden haben kann. Umgekehrt sind Rückschlüsse aus den digitalisierten Signalen 21-24 auf fehlerhafte Hirnstromsignale ebenfalls möglich.

Somit ist bei einer Kombination der Sende-/Empfangseinrichtung 2 mit der Hirnstrom-Meßvorrichtung 7 eine Verminderung der Fehlerhäufigkeit beim Erfassen der motorischen Unruheereignisse ermöglicht. Es handelt sich hierbei um eine verbesserte Ausführungsform der Vorrichtung zum Erfassen motorischer Unruheereignisse einer Versuchsperson 1.

In Verbindung mit dem Messen von Hirnströmen werden von der Versuchsperson 1 in der Regel Ruhephasen und stimulierte Aktivitätsphasen in vorgegebener Weise durchlaufen. Stimulierte Aktivitätsphasen umfassen beispielsweise eine Hyperventilation der Versuchsperson 1. Zum Stimulieren der Aktivitätsphasen ist eine Fotosimulationlampe 11 (vgl. Figur 1) vorgesehen, die an die Steuer- und Auswertevorrichtung 4 gekoppelt ist. Bei der Fotosimulationslampe handelt es sich zweckmäßig um eine übliche zum Aussenden von Licht, die im Impulsbetrieb betrieben werden kann. Es hat sich gezeigt, daß die in Verbindung mit der Hirnstrommessung vorgegebenen standardisierten Meßbedingungen, insbesondere die vorgeschriebene Abfolge von Ruhephasen und stimulierte Aktivitätsphasen, die Vergleichbarkeit bzw. statistische Genauigkeit der Meßergebnisse zum automatischen Erfassen der Häufigkeit motorischer Unruheereignisse für die Versuchsperson 1 unterstützen, wobei insbesondere die erfaßten Meßergebnisse in den Ruhephasen für die Diagnostik auszuwerten sind. Bei der automatischen Ermittlung der Häufigkeit berücksichtigt die Steuer- und Auswerteeinrichtung 4 automatisch elektronische Informationen über die zeitliche Zuordnung von Ruhephasen und stimulierte Aktivitästphasen während des vorgegebenen Meßzeitraums.

Die Steuer- und Auswertevorrichtung 4, einschließlich der Mikroprozessoreinrichtung 5 kann als getrennte Einheit ausgebildet sein, wie dieses in Figur 1 schematisch mittels der Blockdarstellung gezeigt ist, oder in eine der Meßvorrichtungen, nämlich die Sende-/Empfangseinrichtung 2 oder den Entfernungsmesser 6 oder die Hirnstrom-Meßeinrichtung 7, integriert sein. Die erfaßten Meßsignale und/oder die bei der Auswertung ermittelten Ergebnisse, für die in der beschriebenen Meßanordnung jeweils auch eine Zeitinformation, d.h. insbesondere eine Information über die Uhrzeit und/oder den Tag und/oder die Zeitdauer der Messung, erzeugt werden kann, können auf einer Anzeigeeinrichtung 12 dargestellt werden. Die Anzeigeeinrichtung 12 kann bei einer anderen Ausführungsform mit der Sende-/Empfangseinrichtung 2 oder der Entfernungsmesser 6 oder der Hirnstrom-Meßeinrichtung 7 kombiniert sein. Die Meßvorrichtungen, d.h. die Sende-/Empfangseinrichtung 2 und/oder den Entfernungsmesser 6 und/oder die Hirnstrom-Meßeinrichtung 7, sofern sie bei der jeweiligen Ausführungsform vorgesehen sind, sind als einzelne Geräte vorgesehen oder können miteinander in einem Gerät kombiniert werden. Die Einzelgeräte oder die Gerätekombinationen sind vorzugsweise als tragbare Geräte ausgeführt, was für den Benutzer die Handhabung beim Einsatz in der Diagnostik erleichtert.

Die mit dem Verfahren und der Vorrichtung zum Erfassen der motorischen Unruheereignisse verbundenen Vorteile ergeben sich auch ohne eine Kombination mit dem Entfernungsmesser 6. Zu den wesentlichen Vorteilen gehört es, daß die gesamte Körpermotorik der Versuchsperson 1 erfaßt werden kann. Es werden lediglich reale Bewegungen der Versuchsperson 1 und nicht die Kontraktion der Muskeln erfaßt. Zwischen der genutzten Meßvorrichtung in Form der Sende-/Empfangseinrichtung 2 und dem Körper der Versuchsperson 1 besteht keinerlei Berührungskontakt, welcher den üblichen Bewegungsablauf der Versuchsperson 1 beeinflussen könnte, was zur Verfälschung des Meßergebnisses führen würde. Die beschriebene Meßvorrichtung ist in ihrer Handhabung einfach und deshalb für einen routinemäßigen Einsatz zur Meßwerterfassung geeignet.

## Patentansprüche

1. Verfahren zum automatischen Erfassen motorischer Unruhe einer Versuchsperson (1), wobei das Verfahren die folgenden Schritte aufweist:
- Aussenden von Mikrowellensignalen in einem Sendekegel (3) mittels einer Mikrowellen-Sendeeinrichtung (2) für einen vorbestimmten Zeitraum,
- Empfangen von reflektierten Mikrowellensignalen, die beim Reflektieren der ausgesendeten Mikrowellensignale an einer sich im Bereich des Sendekegels (3) befindenden Versuchsperson gebildet werden, mittels einer Mikrowellen-Empfangseinrichtung (2),
- automatisches Ermitteln von Frequenz-Dopplerverschiebungen zwischen den ausgesendeten Mikrowellensignalen und reflektierten Mikrowellensignalen infolge motorischer Unruheereignisse der Versuchsperson (1) mittels einer Doppler-Auswerteeinrichtung (10) und
- automatisches Ermitteln einer Häufigkeit des Auftretens motorischer Unruheereignisse in dem vorbestimmten Zeitraum in Abhängigkeit von der ermittelten Frequenz-Dopplerverschiebungen mittels einer Auswertevorrichtung (4),
**dadurch gekennzeichnet, daß** zumindest während eines Teils des vorbestimmten Zeitraums mit Hilfe einer Hirnstrom-Meßvorrichtung (7) eine Hirnstrommessung bei der Versuchsperson (1) ausgeführt wird, wobei von der Hirnstrom-Meßvornchtung (7) erfaßte Hirnstromsignale automatisch mit den empfangenen reflektierten Mikrowellensignalen sowie den ermittelten Frequenz-Dopplerverschiebungen elektronisch zeitlich korreliert werden.

2. Verfahren nach Anspruch 1, **dadurch gekennzeichnet, daß** zeitlich korreliert zu dem Aussenden der Mikrowellensignale mit Hilfe einer Entfernungsmeßeinrichtung (6) wiederholt eine Messung der Entfernung zwischen der Versuchsperson (1) und der Entfernungsmeßeinrichtung (6) und/oder Objekten im Bereich des Sendekegels (3) und der Entfernungsmeßeinrichtung (6) ausgeführt wird.

3. Verfahren nach Anspruch 2, **dadurch gekennzeichnet , daß** zum Messen der Entfernung mit Hilfe der Entfernungsmeßeinrichtung (6) weitere Mikrowellensignale oder optische Signale ausgesendet und reflektierte Teile der ausgesendeten weiteren Mikrowellensignale/optischen Signale empfangen und ausgewertet werden.

4. Verfahren nach einem der vorangehenden Ansprüche, **dadurch gekennzeichnet, daß** die reflektierten Mikrowellensignale während des vorbestimmten Zeitraums im Verlauf von Ruhephasen und von stimulierten Aktivitätsphasen der Versuchsperson (1) erfaßt werden, wobei elektronische Informationen über einen Beginn und ein Ende der Ruhephasen und der stimulierte Aktivitätsphasen von der Auswertevorrichtung (4) automatisch erfaßt werden.

5. Verfahren nach Anspruch 4, **dadurch gekennzeichnet, daß** zum Stimulieren der Aktivitätsphasen Lichtsignale mit Hilfe einer Fotostimulationslampe ausgesendet werden.

6. Verfahren nach einem der vorangehenden Ansprüche, **dadurch gekennzeichnet, daß** mit Hilfe der Auswertevorrichtung (4) unter Berücksichtigung der zeitlichen Korrelation mittels der erfaßten Hirnstromsignale eine Fehlerkorrektur der ermittelten Häufigkeit des Auftretens der motorischen Unruheereignisse in dem vorbestimmten Zeitraum automatisch ausgeführt wird.

7. Verfahren nach Anspruch 6, **dadurch gekennzeichnet, daß** mit Hilfe der Auswertevorrichtung (4) unter Berücksichtigung der zeitlichen Korrelation mit Hilfe der automatisch ermittelten Frequenz-Dopplerverschiebungen einer Fehlerkorrektur der er-faßten Hirnstromsignale eine Fehlerkorrektur automatisch ausgeführt wird.

8. Vorrichtung zum automatischen Erfassen motorischer Unruhe einer Versuchsperson (1), wobei die Vorrichtung die folgenden Merkmale aufweist:
- eine Mikrowellen-Sendeeinrichtung (2) zum Aussenden von Mikrowellensignalen in einem Sendekegel (3) für einen vorbestimmten Zeitraum,
- eine Mikrowellen-Empfangseinrichtung (2) zum Empfangen von reflektierten Mikrowellensignalen, die beim Reflektieren der ausgesendeten Mikrowellensignale an einer sich im Bereich des Sendekegels (3) befindenden Versuchsperson (1) gebildet werden,
- eine Doppler-Auswerteeinrichtung (10) zum automatischen Ermitteln von Frequenz-Dopplerverschiebungen zwischen den ausgesendeten Mikrowellensignalen und reflektierten Mikrowellensignalen infolge motorischer Unruheereignisse der Versuchsperson (1) und
- eine Auswertevorrichtung (4) zum automatischen Ermitteln einer Häufigkeit des Auftretens motorischer Unruheereignisse in Abhängigkeit von der ermittelten Frequenz-Dopplerverschiebungen in dem vorbestimmten Zeitraum,
**dadurch gekennzeichnet, daß** die Mikrowellen-Sendeeinrichtung und die Mikrowellen-Empfangseinrichtung zum zeitlichen Abstimmen des Aussendens der Mikrowellensignale und des Empfangens der reflektierten Mikrowellensignale mit einer Hirnstrommessung bei der Versuchsperson (1) mit Hilfe einer Hirnstrom-Meßvorrichtung (7) an eine gemeinsame Zeitgebereinheit gekoppelt sind.

9. Vorrichtung nach Anspruch 8, **gekennzeichnet durch** eine lokale Speichereinrichtung zum elektronischen Speichern von Informationen über die ermittelten Doppler-Frequenzverschiebungen und/oder die ermittelte Häufigkeit des Auftretens der motorischen Unruheereignisse.

10. Vorrichtung nach Anspruch 8 oder 9, **dadurch gekennzeichnet , daß** die Vorrichtung als ein tragbares Handgerät ausgebildet ist.

11. Vorrichtung nach einem der Ansprüche 8 bis 10, **dadurch gekennzeichnet , daß** die Mikrowellen-Sendeeinrichtung und die Mikrowellen-Empfangseinrichtung in eine Sende-/Empfangseinrichtung (2) integriert sind.

12. Vorrichtung nach einem der Ansprüche 8 bis 11, **dadurch gekennzeichnet , daß** an die Hirnstrom-Meßvorrichtung (7) eine Fotostimulationslampe zum Aussenden von Lichtsignalen gekoppelt ist.

13. Verwendung einer Vorrichtung nach einem der Ansprüche 8 bis 12 zum automatischen Erfassen motorischer Unruheereignisse einer Versuchsperson gemäß einem Verfahren nach einem der Ansprüche 1 bis 7.

## Claims

1. Method for the automatic detection of motor super-activity in a test person (1) with the method comprising the following steps:
- Transmitting microwave signals in a transmitting cone (3) by means of a microwave transmission means (2) for a specified period of time:
- Receiving reflected microwave signals that are formed in the reflection of the transmitted microwave signals on a test person located in the area of the transmitting cone (3), by means of a microwave receiving means (2);
- Automatic determination of Doppler frequency shifts between the transmitted microwave signals and reflected microwave signals resulting from motor super-activity events in the test person (1) by means of a Doppler evaluation means (10); and
- Automatic determination of a frequency of occurrence of motor super-activity events in the specified time period depending on the determined Doppler frequency shifts, by means of an evaluation means (4), **characterized in that** at least during a part of the specified time period, a measurement of the test person's brain waves is performed with the help of a brain wave measuring device (7), with an electronic timely correlation of the brain wave signals detected by the brain wave measuring device (7) being performed automatically for the received reflected microwave signals as well as the determined Doppler frequency shifts.

2. Method in accordance with Claim 1, **characterized in that** in timely correlation to the transmitting of the microwave signals, a repeated measurement of the distance between the test person (1) and the distance measuring means (6) and/or objects in the area of the transmitting cone (3) and the distance measuring means (6) is performed with the help of a distance measuring means (6).

3. Method in accordance with claim 2, **characterized in that** additional microwave signals or optical signals are transmitted to measure the distance with the help of a distance measuring means (6), and reflected parts of the transmitted additional microwave signals or optical signals are received and evaluated.

4. Method in accordance with one of the preceding claims, **characterized in that** the reflected microwave signals are detected during the specified time period in the course of resting phases and of stimulated activity phases of the test person (1), with electronic information about a start and an end of the resting phases and the stimulated activity phases being recorded automatically by the evaluation device (4).

5. Method in accordance with Claim 4, **characterized in that** light signals are transmitted with the help of a photo stimulation lamp to stimulate the activity phases.

6. Method in accordance with one of the preceding Claims, **characterized in that** with the help of the evaluation device (4) and taking into account the timely correlation by means of the detected brain wave signals, an error correction of the determined frequency of occurrence of the motor super-activity events during the specified time period is performed automatically.

7. Method in accordance with Claim 6, **characterized in that** with the help of the evaluation device (4) and taking into account the timely correlation, an error correction is performed automatically with the help of the automatically determined Doppler frequency shifts of an error correction of the detected brain wave signals.

8. Device for the automatic detection of motor super-activity in a test person (1), with the device having the following characteristics:
- a microwave transmitting means (2) to transmit microwave signals in a transmitting cone (3) for a specified time period;
- a microwave receiving means (2) to receive reflected microwave signals that are formed when the transmitted microwave signals reflect on a test person (1) located in the range of the transmitting cone (3);
- a Doppler evaluation means (10) for the automatic determination of Doppler shifts between the transmitted microwave signals and reflected microwave signals as a result of motor super-activity events in the test person (1); and
- an evaluation device (4) for the automatic determination of a frequency of the occurrence of motor super-activity events depending on the Doppler frequency shifts determined during the specified time period, **characterized in that** the microwave transmitting means and the microwave receiving means for the timely coordination of the transmitting of the microwave signals and the receiving of the reflected microwave signals with a brain wave measurement in the test person (1) with the help of a brain wave measuring device (7) are coupled to a common interval timer unit.

9. Device in accordance with claim 8, **characterized by** a local memory for the electronic storage of information related to the determined Doppler frequency shifts and/or the determined frequency of the occurrence of the motor super-activity events.

10. Device in accordance with claim 8 or 9, **characterized in that** the device is developed as a portable handheld device.

11. Device in accordance with one of Claim 8 to 10, **characterized in that** the microwave transmitting means and the microwave receiving means are integrated in one transmitting- / receiving means (2);

12. Device in accordance with one of the Claims 8 to 11, **characterized in that** a photo stimulation lamp for transmitting light signals in coupled to the brain wave measuring device (7).

13. Use of a device in accordance with one of the Claims 8 to 12 for the automatic detection of motor super-activity events in a test person in accordance with a method according to one of the claims 1 to 7.

## Revendications

1. Procédé pour la saisie automatique de l'agitation motrice d'un sujet d'expérience (1), lequel procédé présente les étapes suivantes :
- émission, au moyen d'un équipement d'émission de micro-ondes (2), de signaux micro-ondes dans un cône d'émission (3) pendant un laps de temps prédéfini,
- réception, au moyen d'un équipement de réception de micro-ondes (2), de signaux micro-ondes réfléchis qui sont formés lors de la réflexion des signaux micro-ondes émis sur un sujet d'expérience se trouvant dans la région du cône d'émission (3),
- détermination automatique, au moyen d'un dispositif d'évaluation Doppler (10), de déplacements de fréquence Doppler entre les signaux micro-ondes émis et les signaux micro-ondes réfléchis à la suite d'événements d'agitation motrice du sujet d'expérience (1) et
- détermination automatique, au moyen d'un dispositif d'évaluation (4), d'une fréquence d'apparition d'événements d'agitation motrice dans le laps de temps prédéfini en fonction des déplacements de fréquence Doppler déterminés,
**caractérisé en ce que**, au moins pendant une partie du laps de temps prédéfini, on effectue à l'aide d'un dispositif de mesure de courant cérébral (7) une mesure de courant cérébral sur le sujet d'expérience (1), les signaux de courant cérébral saisis par le dispositif de mesure de courant cérébral (7) étant automatiquement corrélés électroniquement dans le temps avec les signaux micro-ondes réfléchis reçus ainsi qu'avec les déplacements de fréquence Doppler déterminés.

2. Procédé selon la revendication 1, **caractérisé en ce que**, par corrélation dans le temps avec l'émission des signaux micro-ondes, on effectue de manière répétée à l'aide d'un dispositif de mesure de distance (6) une mesure de la distance entre le sujet d'expérience (1) et l'équipement de mesure de distance (6) et/ou d'objets dans la région du cône d'émission (3) et l'équipement de mesure de distance (6).

3. Procédé selon la revendication 2, **caractérisé en ce que** , pour mesurer la distance à l'aide de l'équipement de mesure de distance (6), d'autres signaux micro-ondes ou signaux optiques sont émis et des parties réfléchies des signaux micro-ondes / optiques émis sont reçues et évaluées.

4. Procédé selon l'une quelconque des revendications précédentes, **caractérisé en ce que** les signaux micro-ondes réfléchis pendant le laps de temps prédéfini sont saisis au cours de phases de repos et de phases d'activité stimulées du sujet d'expérience (1), des informations électroniques sur un début et une fin des phases de repos et des phases d'activité stimulées étant saisies automatiquement par le dispositif d'évaluation (4).

5. Procédé selon la revendication 4, **caractérisé en ce que**, pour stimuler les phases d'activité, des signaux lumineux sont émis à l'aide d'une lampe de photostimulation.

6. Procédé selon l'une quelconque des revendications précédentes, **caractérisé en** c e qu'à l'aide du dispositif d'évaluation (4), en tenant compte de la corrélation temporelle au moyen des signaux de courant cérébral saisis, une correction d'erreur de la fréquence déterminée d'apparition des événements d'agitation motrice dans le laps de temps prédéfini est automatiquement effectuée.

7. Procédé selon la revendication 6, **caractérisé en ce que**, à l'aide du dispositif d'évaluation (4), en tenant compte de la corrélation temporelle à l'aide des déplacements de fréquence Doppler déterminés automatiquement, d'une correction d'erreur des signaux de courant cérébral saisis, une correction d'erreur est automatiquement effectuée.

8. Dispositif pour la saisie automatique de l'agitation motrice d'un sujet d'expérience (1), lequel dispositif présente les caractéristiques suivantes :
- un équipement d'émission de micro-ondes (2) pour l'émission de signaux micro-ondes dans un cône d'émission (3) pendant un laps de temps prédéfini,
- un équipement de réception de micro-ondes (2) pour la réception de signaux micro-ondes réfléchis qui sont formés lors de la réflexion des signaux micro-ondes émis sur un sujet d'expérience (1) se trouvant dans la région du cône d'émission (3),
- un équipement d'évaluation Doppler (10) pour la détermination automatique de déplacements de fréquence Doppler entre les signaux micro-ondes émis et les signaux micro-ondes réfléchis à la suite d'événements d'agitation motrice du sujet d'expérience (1) et
- un dispositif d'évaluation (4) pour la détermination automatique d'une fréquence d'apparition d'événements d'agitation motrice en fonction des déplacements de fréquence Doppler déterminés dans le laps de temps prédéfini,
**caractérisé en ce que** l'équipement d'émission de micro-ondes et l'équipement de réception de micro-ondes sont couplés à une unité horloge commune pour accorder dans le temps l'émission des signaux micro-ondes et la réception des signaux micro-ondes réfléchis avec une mesure de courant cérébral sur le sujet d'expérience (1) à l'aide d'un dispositif de mesure de courant cérébral (7).

9. Dispositif selon la revendication 8, **caractérisé par** r un équipement d'enregistrement local pour l'enregistrement électronique d'informations sur les déplacements de fréquence Doppler déterminés et/ou la fréquence d'apparition déterminée des événements d'agitation motrice.

10. Dispositif selon la revendication 8 ou 9, **caractérisé en ce que** le dispositif est réalisé sous la forme d'un appareil à main portatif.

11. Dispositif selon l'une quelconque des revendications 8 à 10, **caractérisé en ce** q u e l'équipement d'émission de micro-ondes et l'équipement de réception de micro-ondes sont intégrés dans un équipement émetteur/récepteur (2).

12. Dispositif selon l'une quelconque des revendications 8 à 11, **caractérisé en ce qu'**une lampe de photostimulation pour émettre des signaux lumineux est couplée au dispositif de mesure de courant cérébral (7).

13. Utilisation d'un dispositif selon l'une quelconque des revendications 8 à 12 pour la saisie automatique d'événements d'agitation motrice d'un sujet d'expérience conformément à un procédé selon l'une quelconque des revendications 1 à 7.
